# EUROPEAN PATENT APPLICATION

(11) **EP 3 513 783 A1**
(43) Date of publication of application: **24.07.2019**
(21) Application number: 18152498.4
(22) Date of filing: 19.01.2018
(51) Int. Cl.: A61K 9/08, A61K 47/36, A61K 31/4468, A61P 29/02

(54) **CONTAINER FOR PHARMACEUTICAL COMPOSITIONS COMPRISING FENTANYL**

(71) Applicant: Welding GmbH & Co. KG, 20354 Hamburg (DE)
(72) Inventor: Stolte, Sven, 20354 Hamburg (DE); Kemken, Jens, 20354 Hamburg (DE); Friebel, Christian, 20354 Hamburg (DE)
(74) Representative: Hoffmann Eitle

(57) **Abstract**

The present invention relates to a pharmaceutical composition in a container, wherein the container comprises cycloolefin polymer in its main contact surface that is in contact with the pharmaceutical composition, and wherein the pharmaceutical composition comprises a solution of fentanyl or of a pharmaceutically acceptable salt thereof in a solvent; the present invention further relates to a pharmaceutical composition for use in a method for treating pain; and to a drug delivery device containing the pharmaceutical composition.

## Description

The present invention relates to a pharmaceutical composition in a container; to a pharmaceutical composition for use in a method for treating pain; and to a drug delivery device containing the pharmaceutical composition.

Fentanyl is a well-known pharmaceutical ingredient in medications for treating pain and a common route of administration of solutions of fentanyl and its pharmaceutically acceptable salts is by intra nasal administration. The nasal route allows for a rapid pharmaceutical effect and, therefore, is the route of choice for the treatment of acute pain.

Prior art drug delivery devices often contain a container that is made of plastic material. Compared with glass containers, they have the benefit of light weight and shock resistance. Standard packaging materials frequently include polyethylene (PE), polypropylene (PP) and polyethylene terephthalate (PET), and it would be obvious to use these materials for the manufacture of drug delivery devices for fentanyl (salt) solutions.

Surprisingly, however, the present inventors discovered that the content of the active ingredient of such a fentanyl solution decreases over time during storage in a classical plastic container. Interestingly, it was found that the loss of fentanyl ingredient did not result from any degradation. Instead, it was observed that the fentanyl was absorbed into the container wall material. The result, however, was the same because the fentanyl ingredient that was absorbed into the container wall material was no longer available for the treatment of, e.g. acute pain.

Without wishing to be bound by any particular theory, the present inventors believe that the loss of fentanyl continues until the container wall material reaches saturation. In conclusion, it was considered that the loss of fentanyl ingredient is particularly noteworthy for compositions that have a relatively low concentration of fentanyl (such as less than 0.5 mg/ml). When the starting fentanyl concentration is low, the absorption into the container wall is relatively high because the absorption capacity of the wall material is independent of concentration of the pharmaceutical composition. That is to say, a container that is filled with a fentanyl solution having a high concentration of 10 mg/ml may not lose in relation too much of its active ingredient into the container wall whereas the total loss of fentanyl becomes significant at concentrations of below 0.5 mg/ml.

Without wishing to be bound by any theory, the present inventors believe that the standard packaging material of fentanyl (PP, PE, PET) has a particularly high absorption capacity per surface area. Once the material reaches saturation, the loss of fentanyl into the container wall thus causes a significant problem, specifically, for relatively low concentrations of fentanyl.

Against this background, there was a need for pharmaceutical compositions comprising a solution of fentanyl or a pharmaceutically acceptable salt thereof in a solvent with improved concentration stability. For the purposes of the present invention, improved concentration stability defines the reduced loss of fentanyl material caused by absorption into the surrounding container wall material. Specifically, there was a need for a pharmaceutical composition in a container that provides a solution of the above problem without running the risk of fracture as in the case of glass bottles.

The present inventors discovered that the concentration stability of fentanyl during storage can be significantly improved when the surrounding wall material that comes into contact with the fentanyl solution comprises cycloolefin polymer.

In a first aspect, the present invention thus provides a pharmaceutical composition in a container as defined in claim 1, wherein the container comprises cycloolefin polymer in its main contact surface that is in contact with the pharmaceutical composition.

In a preferred embodiment, the present invention relates to such a pharmaceutical composition wherein the main contact surface of the container is the inner surface of a bag. In a further preferred embodiment, the bag is a flexible bag. Most preferably, the flexible bag helps to deliver the pharmaceutical composition through an opening of the container wherein pressure equalization takes place upon delivery of the pharmaceutical composition without any air entering into the container to replace the expelled dose.

The container that is used for the purposes of the present invention comprises the fentanyl (salt) solution that comes into contact with the inner side of the container. The contact area is referred to throughout this invention as the "main contact surface". This surface represents the contact area of the container that comes into contact with the solution and comprises cycloolefin polymer. Preferably, this surface contains at least 70 wt.%, preferably at least 80 wt.%, even more preferably at least 90 wt.% or more of the cycloolefin polymer. In a further preferred embodiment, the main contact surface entirely consists of the cycloolefin polymer.

The term "wherein the main contact surface consists of cycloolefin polymer" and similar expressions define a container, preferably a bag, wherein the inner layer of or the entire wall of the container is made from a cycloolefin polymer material such that the inner surface area does not contain relevant amounts of other polymer materials such as PE, PET, PP.

The cycloolefin polymer that can be used according to the present invention is a cycloolefin homopolymer or a cycloolefin copolymer. Preferably, the cycloolefin polymer is selected from the group consisting of cycloolefin homopolymers and cycloolefin copolymers comprising at least 50, preferably at least 80, more preferably at least 90 percent by weight (based on the total weight of the polymer) of units derived from one or more cycloolefin(s). Suitable cycloolefin monomers include various cyclic monomers, preferably bicyclic or polycyclic monomers such as norbornene and tetracyclodecene (1,2,3,4,4a,5,8,8a-octahydro-1,4:5,8-dimethanonaphthalene) and their derivatives. Preferably, the monomer is norbornene or a derivative thereof. For the manufacture of preferred cycloolefin copolymers, norbornene can be copolymerized with, e.g. ethene. Preferred cycloolefin homopolymers can be prepared by ring-opening metathesis polymerization reaction of norbornene and its derivatives. Commercially available examples of such cycloolefin polymers are manufactured by ZEON CORPORATION and marketed under the tradename Zeonex® and Zeonor®.

The container can be prepared by standard techniques using cycloolefin polymer as the material for the inner wall of the container. For example, the container can be manufactured as described in EP 0 633 122. This European patent describes the fabrication of a "container" containing a flexible inner bag, wherein the inner bag that is described in this reference corresponds to the container of the present invention. In a preferred embodiment, the container of the present invention is prepared from a three layered film, the inner layer of the container being made from the cycloolefin polymer material described herein above. Preferred containers have an inner volume of from 10 to 50, preferably of from 10 to 30, most preferably of from 10 to 20 ml.

The term "fentanyl" as used herein relates to fentanyl or a pharmaceutically acceptable salt thereof. The expression "fentanyl equivalent" defines the amount of fentanyl free base. A concentration of 0.4 mg/ml of fentanyl would thus correspond to the same concentration of "fentanyl equivalents", whereas the amount of a pharmaceutically acceptable fentanyl salt would differ from the concentration of fentanyl equivalents.

In a preferred embodiment, fentanyl is used in the form of its citrate, but other pharmaceutically acceptable salts of fentanyl, including, e.g. hydrochloride are also suitable.

Typical solvents that are used in the fentanyl solution of the present invention are water, polyethylene glycol, pharmaceutically acceptable alcohol (preferably ethanol) and combinations thereof. If water is used as the solvent, sodium chloride may preferably be added to provide a saline solution of the active material (fentanyl).

In a particularly preferred embodiment, the solution has a concentration of less than 0.5 mg/ml, preferably of from 0.15 to 0.45 mg/ml, most preferably of from 0.3 to 0.4 mg/ml of fentanyl equivalents.

In a preferred embodiment, the container discussed herein is part of a drug delivery device for the administration of the pharmaceutical composition to a mammal in need thereof. The drug delivery device allows the intranasal administration and comprises, in addition to the container of claim 1, a housing and a spray head to discharge the pharmaceutical composition. In a preferred embodiment, the drug delivery device further comprises administration control means such as a lock out interval to avoid a possible overdose. In a most preferred embodiment, the container that forms part of the drug delivery device and that is filled with the pharmaceutical composition is a flexible bag enabling the administration of the pharmaceutical composition in any arbitrary position. For example, the drug delivery device may be used in a horizontal arrangement or turned upside-down (with the spray head pointing downwards) by a mammal when lying.

It has been found that using the container that contacts the pharmaceutical composition with a main contact surface that comprises cycloolefin polymer surprisingly avoids a loss of active pharmaceutical ingredient (fentanyl) into the container wall. Without wishing to be bound by any theory, the inventors believe that this observation is particularly valuable for solutions of fentanyl that have a relatively low concentration of, e.g. below 0.5 mg/ml of fentanyl equivalents, at least because a loss of the active pharmaceutical ingredient (fentanyl) into the container wall makes up a significant proportion for such solutions that is no longer available for the treatment of, e.g. pain.

In a further embodiment, the present invention thus relates to a pharmaceutical composition for use in a method of treating pain. In a further preferred embodiment, the method is a method for treating postoperative and/or breakthrough pain. In both cases, i.e. in the treatment of postoperative and/or breakthrough pain, common therapies involve frequent dosages of fentanyl solutions that have a relatively low concentration.

The pharmaceutical composition discussed herein can be prepared by dissolving fentanyl or its pharmaceutically acceptable salt and, optionally, further pharmaceutically acceptable excipients in the solvent. Examples of pharmaceutically acceptable excipients include, without limitation, thickeners such as sodium hyaluronate; buffering agents such as sodium dihydrogen phosphate dihydrate and disodium phosphate; and sodium chloride that helps to adjust the osmolality of the pharmaceutical composition.

A preferred example of the pharmaceutical composition consists of [the below amounts indicating the amount per milliliter pharmaceutical composition]:

| | |
|---|---|
| 0.561 mg | fentanyl citrate |
| 0.100 mg | sodium hyaluronate |
| 1.000 mg | sodium dihydrogen phosphate dihydrate |
| 2.750 mg | disodium phosphate, anhydrous |
| 7.770 mg | sodium chloride |
| 994.019 mg | water |

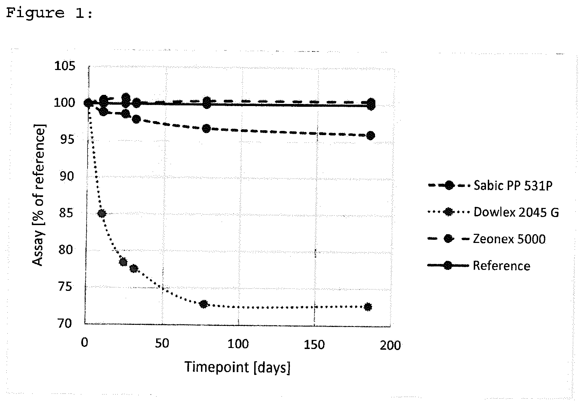

Figure 1 compares the concentration of a fentanyl citrate solution in aqueous buffer solution pH 7.0 over time at 40°C in contact to different container materials. The materials employed in this study were: Sabic® PP 531, a polypropylene that is commercially available from Saudi Basic Industries Coporation; Dowlex 2045 G, a polyethylene commercially available from Dow; Zeonex® 5000, a cycloolefin polymer commercially available from ZEON; and a standard glass vial.

As can be seen from the data presented in the accompanying figure, no fentanyl is absorbed into the container wall material over time as when the container is made of glass (reference). Importantly, no loss of fentanyl ingredient is observed for the cycloolefin polymer material as the fentanyl concentration at the different time points overlap with the reference material (glass). The use of standard materials such as polypropylene and polyethylene, however, causes a significant loss of available active material. This loss or absorption into the material continues over time and can reach values of as much as 10 % or more over the ordinary life span of such a medicament.

The use of cycloolefin polymer, therefore, is a clear improvement that helps to maintain the concentration of available fentanyl material at the desired therapeutic level.

## Claims

1. Pharmaceutical composition in a container, wherein the container comprises cycloolefin polymer in its main contact surface that is in contact with the pharmaceutical composition, and wherein the pharmaceutical composition comprises a solution of fentanyl or of a pharmaceutically acceptable salt thereof in a solvent.

2. Pharmaceutical composition according to claim 1, wherein the main contact surface of the container is the inner surface of a bag.

3. Pharmaceutical composition according to claim 2, wherein the bag is a flexible bag.

4. Pharmaceutical composition according to any of the preceding claims, wherein the main contact surface consists of cycloolefin polymer.

5. Pharmaceutical composition according to any of the preceding claims, wherein the cycloolefin polymer is a cycloolefin copolymer.

6. Pharmaceutical composition according to any of the preceding claims, wherein the pharmaceutically acceptable salt of fentanyl is fentanyl citrate and/or fentanyl hydrochloride.

7. Pharmaceutical composition according to any of the preceding claims, wherein the solvent is water or polyethylene glycol or a pharmaceutically acceptable alcohol or a mixture of any of these.

8. Pharmaceutical composition according to any of the preceding claims, wherein the solution has a concentration of less than 0.5 mg/ml of fentanyl equivalents.

9. Pharmaceutical composition according to any of the preceding claims, wherein the container is part of a drug delivery device for the administration, preferably for the intranasal administration, of the pharmaceutical composition to a mammal in need thereof.

10. Pharmaceutical composition according to claim 9, wherein the drug delivery device further comprises, in addition to the container, a housing and a spray head to discharge the pharmaceutical composition.

11. Pharmaceutical composition according to claim 10, wherein the drug delivery device further comprises administration control means.

12. Pharmaceutical composition according to any of the preceding claims for use in a method of treating pain comprising administering the pharmaceutical composition to a mammal in need thereof.

13. Pharmaceutical composition for use according to claim 12, wherein the method is a method for treating postoperative pain and/or breakthrough pain.

14. Drug delivery device containing a pharmaceutical composition comprising a solution of fentanyl or of a pharmaceutically acceptable salt thereof in a solvent, wherein the drug delivery device comprises a housing, a container filled with said solution and a spray head to discharge said solution, and wherein the main contact surface of the container that is in contact with said solution comprises cycloolefin polymer.

15. Pharmaceutical composition according to any of claims 1 to 11, or pharmaceutical composition for use according to any of claims 12 or 13, or drug delivery device according to claim 14, wherein the pharmaceutical composition is prepared by dissolving fentanyl or its pharmaceutically acceptable salt and, optionally, further pharmaceutically acceptable excipients in the solvent.
